Europäisches Patentamt

**European Patent Office**

Office européen des brevets

(19)

(11) Veröffentlichungsnummer: **0 059 297**

**A1**

(12) # EUROPÄISCHE PATENTANMELDUNG

(21) Anmeldenummer: **81890181.1**

(22) Anmeldetag: **23.10.81**

(51) Int. Cl.³: **A 61 B 5/14**
**B 01 L 3/14**

(30) Priorität: **27.02.81 AT 916/81**

(43) Veröffentlichungstag der Anmeldung:
**08.09.82 Patentblatt 82/36**

(84) Benannte Vertragsstaaten:
**BE CH DE FR GB IT LI LU NL SE**

(71) Anmelder: **C.A. GREINER & SÖHNE GESELLSCHAFT**
**M.B.H.**
**Greinerstrasse 70**
**A-4550 Kremsmünster Oberösterreich(AT)**

(72) Erfinder: **Konrad, Franz**
**in Preising 106**
**A-4844 Regau Oberösterreich(AT)**

(74) Vertreter: **Krause, Ernst, Dipl.-Ing. et al,**
**Dipl.-Ing. Krause Ernst Dipl. Ing. Casati Wilhelm**
**Patentanwälte Amerlingstrasse 8**
**A-1061 Wien(AT)**

(54) **Mit einer Dichtung verschlossenes, evakuierbares Blutprobenröhrchen.**

(57) Mit einer Dichtung verschlossenes, evakuierbares Blutprobenröhrchen, Verpackung hiefür sowie Verfahren zur Herstellung dieser Verpackung.

Ein mit einer Dichtung verschlossenes, evakuierbares Blutprobenröhrchen (1), bestimmt zur Verwendung in einem Blutabnahmegerät mit einem hohlzylindrischen Halter, der einenends eine doppelendige Hohlnadel aufweist und in dessen anderes, offenes Ende das Blutprobenröhrchen soweit einschiebbar ist, daß das eine Nadelende die Dichtung durchstößt, ist an seinem offenen Ende durch einen Verschluß (2) abgedeckt, der außen die Dichtung (6) aus gummielastischem Werkstoff trägt; ein heißschmelzendes Material ist zwischen dem Rohrende und dem Verschluß (2) angeordnet. Zwecks besonders einfacher Herstellung und Handhabung des Röhrchens besteht der Verschluß (2) aus einer ebenen, vorzugsweise gleichbleibende Wandstärke aufweisenden Folie, die einseitig mit einer durchgehenden heißsiegelbaren Schicht (3) versehen ist, welche eine dichte Verbindung zwischen der Folie und der Endstirnfläche des Röhrchens (1) bildet und den Röhrcheninnenraum gegen die Folie isoliert.

Eine Verpackung für solche Röhrchen besteht aus einer das bzw. die Röhrchen (1) aufnehmenden, insbesondere durch eine Schweißnaht (9) verschlossenen Umhüllung (8) aus einem luftdichten Kunststoff, wobei diese Umhüllung (8) unter Vakuum gesetzt ist. Zur Herstellung dieser Verpackung wird so verfahren, daß unter Vakuum gesetzte Röhrchen in die Umhüllung eingebracht werden und ein vorbestimmtes Vakuum in der Umhüllung erzeugt wird, worauf die Umhüllung verschlossen wird.

FIG.1

EP 0 059 297 A1

0059297

<u>Mit einer Dichtung verschlossenes, evakuierbares Blut-</u>
<u>probenröhrchen</u>

Die Erfindung bezieht sich auf ein mit einer Dichtung verschlossenes, evakuierbares Blutprobenröhrchen
für ein Blutabnahmegerät mit einem hohlzylindrischen
Halter, der einenends eine doppelendige Hohlnadel aufweist und in dessen anderes, offenes Ende das Blutprobenröhrchen soweit einschiebbar ist, daß das eine Nadelende die Dichtung durchstößt, wobei das Röhrchen an seinem
offenen Ende durch einen Verschluß abgedeckt ist, der
außen die Dichtung aus gummielastischem Werkstoff trägt,
unter Anordnung eines heißschmelzenden Materials zwischen
dem Rohrende und dem Verschluß.

Ein Röhrchen für ein Vakuum-Blutentnahmesystem ist
aus der DE-OS 29 08 817 bekannt. Den Verschluß bildet
dort eine aufschraubbare Kappe, die eine Öffnung aufweist, deren Rand eine die Dichtung bildende Membrane
aus einem Material, das vom einen Ende der Nadel durchstechbar ist, an das offene Ende des Blutprobenröhrchens
abdichtend andrückt. Bei diesem Röhrchen müssen also an
seiner Öffnung und an seiner Verschlußkappe Gewinde hergestellt werden, und ferner muß beim Aufschrauben der
Kappe eine sorgfältige Dichtung der Membrane zwischen der
Kappe und dem Röhrchenende hergestellt werden. Der Zugang
zu der mit einem solchen Röhrchen entnommenen Probe erfordert letztlich wieder das Abschrauben der Kappe, was
umständlich und zeitraubend ist; außerdem kann am Röhrchen nicht festgestellt werden, ob ein ungewolltes Öffnen vorgenommen ist. Außerdem hat sich herausgestellt,
daß bei längerer Lagerung dieser Röhrchen, insbesondere
wenn sie aus mikroporösem Kunststoff bestehen, das Vakuum nicht mehr oder nur mehr ungenügend vorhanden ist.

Ein Röhrchen der eingangs genannten Art ist aus
der DE-OS 22 43 593 bekannt. Bei einer besonderen Ausführungsform wird dabei eine nach Art eines Kronenverschlusses geformte Metallkappe vorgesehen, die in ihrem Mittelteil einen kreisförmigen Kronenteil und daran nach außen anschließend eine ringförmige herabhängende Manschette mit umgekehrtem U-Profil aufweist, das in einen nach oben umgekröpften Rand endet. Der Mittelteil des kreisförmigen Kronenteiles ist mit geringerer Dicke ausgebildet, um leicht von der Hohlnadel durchstochen werden zu können. Wenigstens die Innenfläche der Metallkappe, vorzugsweise ihre gesamte Unterfläche, ist dabei mit einem dünnen Schutzüberzug aus Vinyl überzogen, um eine Verunreinigung des Röhrcheninhaltes hintanzuhalten. Die Dichtung ist an der Außenfläche der Metallkappe befestigt, und die Festlegung der Kappe mit ihrem U-Profil am Röhrchenende erfolgt mit Hilfe eines heißschmelzenden Materials, wie Polyamid, das eine Klebebindung zwischen der Stoßfläche der Manschette und der Außenfläche des Rohrendes bildet. In dieser Weise kann die Metallkappe mit einem Kronenkorkenöffner abgenommen werden. Auch diese bekannte Lösung weist nicht nur eine Vielzahl von kompliziert geformten bzw. herzustellenden Einzelteilen auf, sondern auch einen umständlichen Aufwand beim Zusammenbau und bei der Handhabung.

Aus der US-PS 4 122 947 ist es bekannt, unter Vakuum gesetzte Blutprobenröhrchen in einem Behälter aus durchsichtigem Kunststoff aufzubewahren und alle Teile mit besonderen Identifikationen auszustatten, um Verwechslungen bezüglich des untersuchten bzw. zu behandelnden Patienten auszuschließen.

Die Erfindung stellt sich die Aufgabe, diese Übelstände zu beseitigen und ein in besonders einfacher Weise herstellbares Röhrchen der eingangs genannten Art, das auch leicht und rasch zu öffnen ist, und eine Verpackung hiefür zu schaffen. Erfindungsgemäß ist dazu vorgesehen, daß der Verschluß aus einer ebenen, vorzugsweise gleichbleibende Wandstärke aufweisenden Folie,besteht, die einseitig mit einer durchgehenden heißsie-

- 3 -

0059297

gelbaren Schicht versehen ist, welche eine dichte Verbindung zwischen der Folie und der Endstirnfläche des Röhrchens bildet und den Röhrcheninnenraum gegen die Folie isoliert. Auf diese Weise wird ein leicht zu öffnender Originalitätsverschluß bewirkt, der auch besonders einfach herzustellen ist.

Eine besonders leicht handzuhabende Ausführungsform sieht vor, daß der Verschluß, der vorzugsweise in an sich bekannter Weise aus einer etwa 0,3 mm starken Aluminiumfolie besteht, mit einem seitlich abstehenden Abziehlappen versehen ist. Vorteilhafterweise ist dabei zur Vergrößerung der Heißsiegelfläche vorgesehen, daß das Röhrchen einen verbreiterten Rand an seiner Öffnung aufweist.

Die Verpackung für ein solches Röhrchen, oder eine Gruppe derselben, ist nach einem weiteren Merkmal der Erfindung dadurch ausgezeichnet, daß sie in an sich bekannter Weise aus einer bzw. das die Röhrchen aufnehmenden, insbesondere durch eine Schweißnaht verschlossenen Umhüllung aus einem luftdichten Kunststoff besteht und daß diese Umhüllung unter Vakuum gesetzt ist. Dadurch läßt sich eine erhöhte Lagerzeit der Röhrchen erzielen.

Zur Herstellung einer solchen Verpackung wird zweckmäßig so verfahren, daß unter Vakuum gesetzte Röhrchen in die Umhüllung eingebracht werden, daß ein vorbestimmtes Vakuum in der Umhüllung erzeugt wird, wobei vorzugsweise ein Gleichgewicht zwischen diesem Vakuum und demjenigen im Röhrchen hergestellt wird, worauf die Umhüllung, vorzugsweise durch Schweißen, verschlossen wird. Eine so hergestellte Verpackung ermöglicht eine besonders lange Lagerung der Röhrchen ohne Verlust an deren Vakuum.

Weitere Einzelheiten und Vorteile der Erfindung ergeben sich aus der folgenden Beschreibung eines Ausführungsbeispieles anhand der Zeichnung; es zeigen:

Fig. 1 eine schematische Seitenansicht des Röhrchens, teilweise im Schnitt,

Fig. 2 eine Draufsicht auf seine Platine aus Aluminiumfolie, und

Fig. 3 eine Seitenansicht einer Verpackung für drei Röhrchen.

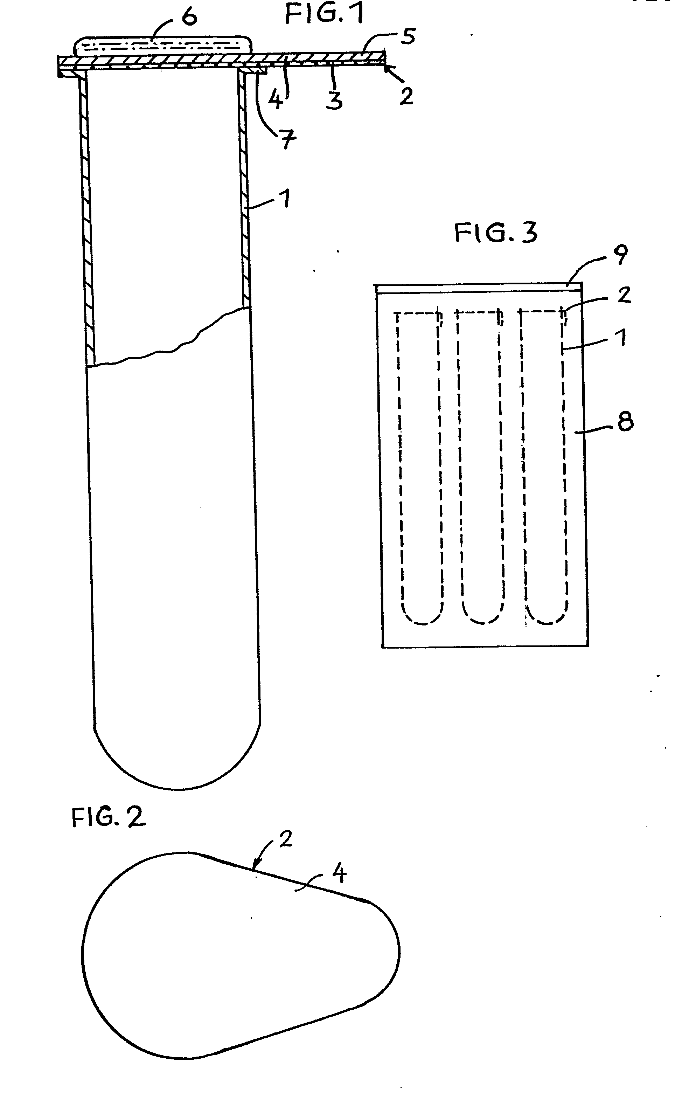

Wie aus der Zeichnung hervorgeht, weist das Probenröhrchen 1, das zweckmäßig aus einem durchsichtigen Kunststoff, wie Polystyrol oder Polypropylen, bestehen kann, an seinem offenen Ende einen Verschluß 2 in Form einer Platine aus einer heißsiegelfähigen, also mit einer Heißsiegelschicht 3 versehenen, gleichbleibende Wandstärke aufweisenden Aluminiumfolie 4 auf. Solche Folien sind an sich bekannt. Wie aus Fig. 2 ersichtlich, besitzt der Verschluß 2 einen seitlich abstehenden Abziehlappen 5, mit dessen Hilfe das Röhrchen geöffnet werden kann, um Zugang zur Probe zu erhalten. Zweckmäßig wird eine 0,3 mm starke Aluminiumfolie verwendet. Es ist dabei vorgesehen, diesen Abreißlappen 5 an die Wand des Röhrchens 1 anzudrücken, damit ein einwandfreies Einschieben des Röhrchens 1 in den (nicht dargestellten) hohlzylindrischen Halter des Blutabnahmegerätes (vgl. DE-OS 29 08 817) ermöglicht wird.

Auf dem Verschluß 2 ist ferner ein etwa 1,5 - 3, z.B. 2 mm starkes Dichtungspolster 6 angeordnet, das aus einem gummielastischen Werkstoff, vorzugsweise Silikonkautschuk, besteht. Dieses luftdicht aufvulkanisierte Dichtungspolster hat die Eigenschaft, daß es sich, wenn man das eine Ende der doppelendigen Nadel des hohlzylindrischen Halters hindurchsticht und die Nadel nach der Blutentnahme wieder herauszieht, wieder ganz schließt. Vorteilhaft ist die Öffnung des Röhrchens 1 mit einem verbreiterten Rand 7 versehen, um die Siegelfläche zu vergrößern.

Der Innenraum des Röhrchens steht unter Vakuum; dieses kann nach dem Heißsiegeln des Verschlusses 2 und dem Aufbringen des Dichtungspolsters 6 erzeugt werden.

Die Handhabung und Wirkungsweise dieses Röhrchens bezüglich der Blutabnahme ist wie in der genannten DE-OS beschrieben, jedoch mit dem Vorteil, daß es durch einfaches Abziehen des Verschlusses 2 mittels des Lappens 5 leicht geöffnet werden kann und daß bei dieser Öffnung durch das seitliche Abziehen kein Aerosoleffekt entsteht, wie dies bei bekannten Geräten (vgl. US-PS 2 460 641 und 3 136 440) der Fall ist.

An dem beschriebenen Ausführungsbeispiel können Abänderungen vorgenommen werden. So kann das Röhrchen 1 z.B. auch aus Glas bestehen. Es ist ferner möglich, den Verschluß 2 auch aus einer Kunststoffolie herzustellen.

Da solche Röhrchen, insbesondere wenn sie aus mikroporösem Kunststoff bestehen, schwierig über längere Zeit unter voller Aufrechterhaltung ihres Vakuums zu lagern sind, wird eine besondere, in Fig. 3 dargestellte Verpackung für ein oder mehrere solche Röhrchen 1 vorgesehen. Diese Verpackung besteht aus einem Beutel 8 aus luftdichtem Kunststoff, insbesondere einer Schrumpffolie, der eine Umhüllung für das bzw. die Röhrchen 1 bildet und nach deren Einbringung ebenfalls unter Vakuum gesetzt und dann verschlossen wird, z.B. durch eine Schweißnaht 9. Das Vakuum im Beutel kann dabei größer als jenes im Röhrchen oder auch so bemessen sein, daß es denselben Wert wie jenes im Röhrchen aufweist, sodaß sich in beiden Fällen letztlich ein Gleichgewichtszustand einzustellen vermag.

Das erfindungsgemäß ausgebildete Röhrchen kann nicht nur zur Blutentnahme, sondern auch zur Entnahme von anderen Körperflüssigkeiten und auch für dazu ähnliche Zwecke eingesetzt werden.

Patentansprüche:

1. Mit einer Dichtung verschlossenes, evakuierbares Blutprobenröhrchen für ein Blutabnahmegerät mit einem hohlzylindrischen Halter, der einenends eine doppelendige Hohlnadel aufweist und in dessen anderes, offenes Ende das Blutprobenröhrchen soweit einschiebbar ist, daß das eine Nadelende die Dichtung durchstößt, wobei das Röhrchen an seinem offenen Ende durch einen Verschluß abgedeckt ist, der außen die Dichtung aus gummielastischem Werkstoff trägt, unter Anordnung eines heißschmelzenden Materials zwischen dem Rohrende und dem Verschluß, dadurch gekennzeichnet, daß der Verschluß(2) aus einer ebenen, vorzugsweise gleichbleibende Wandstärke aufweisenden Folie, besteht, die einseitig mit einer durchgehenden heißsiegelbaren Schicht (3) versehen ist, welche eine dichte Verbindung zwischen der Folie und der Endstirnfläche des Röhrchens (1) bildet und den Röhrcheninnenraum gegen die Folie isoliert.

2. Röhrchen nach Anspruch 1, dadurch gekennzeichnet, daß der Verschluß (2), der vorzugsweise in an sich bekannter Weise aus einer etwa 0,3 mm starken Aluminiumfolie besteht, mit einem seitlich abstehenden Abziehlappen (5) versehen ist.

3. Röhrchen nach Anspruch 1 oder 2, dadurch gekennzeichnet, daß es einen verbreiterten Rand (7) an seiner Öffnung aufweist.

4. Verpackung für ein oder mehrere Röhrchen nach einem der Ansprüche 1 bis 3, dadurch gekennzeichnet, daß sie in an sich bekannter Weise aus einer das bzw. die Röhrchen (1) aufnehmenden, insbesondere durch eine Schweißnaht (9) verschlossenen Umhüllung (8) aus einem luftdichten Kunststoff besteht und daß diese Umhüllung (8) unter Vakuum gesetzt ist.

5. Verfahren zur Herstellung einer Verpackung nach Anspruch 4, dadurch gekennzeichnet, daß unter Vakuum gesetzte Röhrchen in die Umhüllung eingebracht werden,

0059297

daß ein vorbestimmtes Vakuum in der Umhüllung erzeugt wird, wobei vorzugsweise ein Gleichgewicht zwischen diesem Vakuum und demjenigen im Röhrchen hergestellt wird, worauf die Umhüllung, vorzugsweise durch Schweißen, verschlossen wird.

FIG.1

FIG.3

FIG.2

**0059297**

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A | FR-A-2 007 707 (EPPENDORF GERATEBAU NETHELER & HINZ GmbH) * Seite 2, Zeilen 11-31; Seite 3, Zeilen 11-24; Seite 4, Zeilen 4-14; Figur 1 * | 1-3 | A 61 B 5/14 B 01 L 3/14 |
| | --- | | |
| A | US-A-3 992 150 (E. RETZER/COMPUR-WERK GmbH) * Spalte 2, Zeilen 50-66; Figur 1 * | 1 | |
| | --- | | |
| A | US-A-2 876 775 (C.H. BARR et al.) * Spalte 1, Zeile 63 - Spalte 2, Zeile 14; Figur 1 * | 2 | |
| | --- | | |
| D,A | EP-A-0 017 728 (C.A. GREINER & SOHNE GmbH) * Seite 7, Zeile 10 - Seite 8, Zeile 17; Figur 1 * | 1 | |
| | --- | | |
| D,A | DE-A-2 243 593 (CORNING GLASS WORKS) * Seite 6, Zeile 17 - Seite 7, Zeile 9; Figur 3 * | 1 | |
| | --- | | |
| A | FR-A-2 387 042 (BECTON, DICKINSON AND COMPANY) * Seite 4, Zeile 36 - Seite 5, Zeile 28; Figur 2 * | 1 | |
| | --- | | |

**RECHERCHIERTE SACHGEBIETE (Int. Cl. ³)**

```
A 61 B    5/14
B 01 L    3/14
B 65 D   81/20
A 61 G   12/00
```

Der vorliegende Recherchenbericht wurde für alle Patentansprüche erstellt.

| Recherchenort | Abschlußdatum der Recherche | Prüfer |
|---|---|---|
| DEN HAAG | 01-06-1982 | ZILLIOX J.M. |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A | GB-A- 951 228 (IMPERIAL CHEMICAL INDUSTRIES LTD.) * Seite 1, Zeile 22 - Seite 2, Zeile 34 * | 4,5 | |
| | --- | | |
| D,A | US-A-4 122 947 (M.B. FALLA) * Zusammenfassung; Spalte 5, Zeile 38-65; Spalte 4, Zeilen 29-51; Figuren 1,2 * | 5 | |
| | ----- | | |

RECHERCHIERTE SACHGEBIETE (Int. Cl. ³)

Der vorliegende Recherchenbericht wurde für alle Patentansprüche erstellt.

| Recherchenort DEN HAAG | Abschlußdatum der Recherche 01-06-1982 | Prüfer ZILLIOX J.M. |
|---|---|---|